# EUROPEAN PATENT APPLICATION

(11) **EP 1 438 921 A1**
(43) Date of publication of application: **21.07.2004**
(21) Application number: 03025668.9
(22) Date of filing: 07.11.2003
(51) Int. Cl.: A61B 5/06, A61B 19/00

(54) **Device for detecting a position and an orientation of a medical insertion tool inside a body cavity**

(30) Priority: 16.01.2003 JP 2003008812
(71) Applicant: Uchihashi Estec Co., Ltd., Chou-ku, Osaka-shi, Osaka (JP); Ikeuchi, Ken, Kyoto-shi, Kyoto (JP)
(72) Inventor: Toyoda, Kazumi, Uchihashi Estec Co. Ltd., Osaka-shi Osaka (JP); Okazaki, Tomoki, Sakyo-ku, Kyoto-shi Kyoto (JP); Ikeuchi, Ken, Kyoto-shi Kyoto (JP)
(74) Representative: Fleuchaus, Leo, Dipl.-Ing.

(57) **Abstract**

The present invention relates to a device for detecting a position and an orientation of an insertion portion of a medical insertion tool which is inserted inside a body cavity, from the outside, and a detecting method therefor. An object of the present invention can achieve a downsized and lightweight insertion portion so as to be smoothly and easily insertable into even a narrow body cavity such as a blood vessel inside a skull, without having a bad influence on a vital tissue. Moreover, another object thereof is to make it possible to detect the three-dimensional position and orientation of the insertion tool very accurately while minimizing a noise. According to the present invention, a permanent magnet or a ferromagnetic body 4 is attached to an insertion portion 2 of a catheter 1 as an example of the medical insertion tool, which is inserted into the body cavity. On the other hand, outside the body cavity, at least three triaxial MI sensors 5 having triaxial directivity to the magnetic field generated from the permanent magnet or the ferromagnetic body 4 are equally spaced around a scope to be detected. The magnetic field measurement signal processing circuit 6 is connected to the triaxial MI sensors 5.

## Description

### Background of the Invention

The present invention is suitable for a catheter used for performing a non-craniotomy minimally invasive treatment by means of inserting the catheter into a narrow blood vessel without incising a head in mainly a treatment and a diagnosis of brain infarction, a cerebral aneurysm or the like. The present invention relates to a device for detecting a position and an orientation of an insertion portion of a medical insertion tool, which is inserted inside a body cavity, from the outside, and a detecting method therefor. The medical insertion tool is selected from indwelling tools inside the body cavity such as a guide wire, an endoscope or a drainage tube for forming a discharge passage for body fluid, a biliary stent, a high calorie transfusion tube as well as the catheter.

When a narrow tubular medical insertion tool such as the catheter to be inserted into a vein is inserted into a predetermined position inside a body cavity, it is extremely important to exactly detect and grasp the current position and orientation of the medical insertion tool so as to prevent the insertion portion from straying off, adversely affecting a tissue of a living body as a result of a state wherein the insertion portion catches the tissue owing to a friction between the insertion portion and a surface of the tissue of the living body. Here, the orientation means a direction and a twist of a tip of the insertion portion.

As a means for detecting the position and orientation of the medical insertion tool having the above important role, conventionally, an fluoroscopy has been performed, and also a means performed through injection of a contrast medium has been used. However, this requires not only a skillful technique but also contrivance for decreasing anX-rays exposure amount through means that the number of times for fluoroscopy is limited so as to avoid an ill effect on a human body such as a patient or a person to be operated owing to X-rays exposure, or an operator wears a protector accompanying unpleasantness and difficulty in an operation. Moreover, this only provides a two-dimensional image. So, it has a disadvantage wherein the insertion tool is not easily inserted and operated. Especially, the blood vessel inside a skull is narrow, complicatedly wound and has many branches. So, when it is compared with a cardiac catheter of which technique has been substantially established, a treatment and a medical examination through it are difficult and a time-consuming work. Moreover, it should be avoided to expose the head to X-rays. Taking the above circumstances into consideration, it is desired to provide a new method for detecting a position and an orientation thereof instead of fluoroscopy so as to decrease the number of times for fluoroscopy.

As the means for meeting the demand, a research has been developed for detecting a position and an orientation of a target (i.e., an insertion portion of a medical insertion tool to be inserted into a body cavity) by means of the magnetic field. In case of using the magnetic field so as to detect the position and the orientation of the target, a relative permeability of the human body is almost 1, and a distribution of the magnetic field inside the body cavity can be treated in the same way as one in the air. If the magnetic field is set to be an appropriate scale and an appropriate frequency, it has no bad influence on the human body. It has the above usefulness wherein secondary problems do not occur in using the medical insertion tool which is inserted into the body cavity. Noticing the usefulness obtained as a result of use of the magnetic field, a magnetic sensor system for detecting a position and an orientation of the tip of the catheter is proposed as an example of the device whichmakes it possible to detect the three-dimensional position and orientation of the insertion portion of the medical insertion tool. The magnetic sensor system has the following structure: *1*) a tip of a catheter to be used as an example of a medical insertion tool is provided with a magnetic sensor (i.e., a magnetic filed detecting means) such as a triaxial magnet field sensor (i.e., triaxial MI sensor) by exerting amorphous wire magneto-impedance effect elements: *2)* an alternating current magnetic field generated from a biaxial exciting coil (i.e., a magnetic field generating means) disposed outside the body cavity is measured by means of the magnetic sensor disposed on the tip of the catheter: *3)* After a sensor signal is circuit-processed, it is input to a computer through an A/D converter, whereby the three-dimensional position and the orientation of the catheter is sought on the basis of a predetermined algorism by means of the computer in real time: *4)* on the basis of information relating to the position and the orientation thereof, the catheter is superimposed on a three-dimensional image of a blood vessel which was input to the computer by means of CT or MRI, prior to an operation. (cf. Publication E of Conference of the Institute of Electrical Engineers of Japan, 2000 Vol. 120, No. 5 PP.211-218).

However, in the device for detecting a position and an orientation of a medical insertion tool inside a body cavity according to the prior art such as the above-mentioned magnetic sensor system for detecting the position and the orientation of the catheter tip, it is necessary to dispose plural conductors such as a coaxial cable along the insertion portion so as to supply driving electric power for a magnetic sensor to be inserted in the body cavity, which is attachedly disposed on the tip of the insertion portion of the medical insertion tool as the magnetic field detecting means, and fetch a detecting magnetic field signal detected by the magnetic sensor from the inside of the body cavity to the outside thereof. This not only results in an increase of a diameter of the insertion portion, but also easily leads to an increase of a weight thereof. So, this makes it further difficult to insert the insertion portion inside the body cavity such as a blood vessel in a skull, as compared with the prior art. It further aggravates a problem of adversely affecting a tissue of a living body as a result of a state wherein the insertion portion catches the tissue owing to a friction between the insertion portion and a surface of the tissue of the living body.

Inaddition, ithasanotherproblemwhereinanoiseoccurs or is mixed when electric power is supplied to the inside of the body cavity and the detectingmagnetic field signal is fetched from the body cavity. Consequently, S/N ration is reduced, thereby making it impossible to obtain a high detecting accuracy.

### Summary of the Invention

The present invention has been conducted in view of the above-discussed circumstances and problems. An object of the present invention is to provide a small-size and light weight insertion portion so as to make it possible to be smoothly and easily inserted into even a narrow body cavity such as a blood vessel inside a skull, without having a bad influence on a vital tissue. Moreover, another object thereof is to provide the device for detecting the position and the orientation of the medical insertion tool inside the body cavity which makes it possible to detect the three-dimensional position and orientation of the insertion tool very accurately while minimizing a noise and detecting method therefor.

In order to achieve the above obj ects, a device embodying the present invention for detecting a position and an orientation of an insertion portion of a medical insertion tool inside a body cavity by means of a magnetic field generating means and a magnetic field detecting means is characterized in that the magnetic field generating means is attached to the insertion portion of the medical insertion tool; the magnetic field generating means is made of a permanent magnet or a ferromagnetic body which can generate a magnetic field without applying an electric current to a conductor; and the magnetic field detecting means is disposed outside the body cavity, and the magnetic field detecting means including plural magnetic sensors having triaxial directivity to the magnetic field to be detected, each of the magnetic sensors having triaxial directivity being formed by combining plural sensors respectively having uniaxial directivity.

A method embodying the present invention of detecting a position and an orientation of an insertion portion of a medical insertion tool inside a body cavity by means of a magnetic field generating means and a magnetic field detecting means comprises the steps of generating a magnetic field through the magnetic field generating means made of a permanent magnet or a ferromagnetic body attached to the insertion portion, without applying an electric current to a conductor, in a state wherein the insertion portion thereof is inserted inside the body cavity, measuring the generated magnetic field by means of the plural magnetic sensors having the triaxial directivity to the magnetic field to be detected, which are disposed outside the body cavity, each of the magnetic sensors having triaxial directivity being formed by combining plural sensors respectively having uniaxial directivity and detecting a three dimensional position and a three dimensional orientation of the insertion portion of the medical insertion tool.

The present invention having the above elements is a device for detecting a three-dimensional position and orientation of the insertion portion by means of measuring the magnetic field through the magnetic sensor disposed outside the body cavity. The magnetic field is distributed in the air in the almost same way as that of the inside of the body cavity, and is generated by the permanent magnet or the ferromagnetic body attached to the insertion portion of the medical insertion tool to be inserted into the body cavity. So, the present invention does not require disposing a conductor for supplying a sensor driving power and a conductor for taking out a detecting magnetic field signal such as a coaxial cable, along the insertion portion of the medical insertion tool, which is different from the prior art. This can achieve a downsized and lightweight insertion portion of the medical insertion tool. As a result, it can be smoothly and easily performed to insert the insertion portion into even the narrow body cavity such as the blood vessel inside the skull, without having a bad influence on a vital tissue.

Moreover, it is not necessary to apply an electric current to the insertion portion of the medical insertion tool to be inserted in the body cavity. This can make S/N ratio great while minimizing a magnetic field noise which is a cause of a measurement error of the magnetic sensor. In addition, in case that a distance between the permanent magnet or the ferromagnetic body and the magnetic sensor is long, or in case that the permanent magnet or the ferromagnetic body has a specific orientation, the magnetic field to be detected is reduced, and the magnetic noise becomes relatively greater, thereby increasing the measurement error. However, in the present invention, plural magnetic sensors are disposed, the magnetic sensors have triaxial directivity to the magnetic field to be detected. From among the plural magnetic sensors, we select only the sensors located in places where a distance between the permanent magnet or the ferromagnetic body and the magnetic sensor is short, and where the influence of the orientation is less and the magnetic field to be detected is great. Then, the magnetic signals measured by the abovementioned sensor are selectively used. Consequently, the influence of the distance and the orientation can be minimized to decrease the measurement error, thereby making it possible to detect the three-dimensional position and orientation of the insertion portion of the predetermined medical insertion tool accurately.

Especially, three or more magnetic sensors, which work as the magnetic field detecting means in the present invention, are equally spaced around a scope to be detected, thereby making it possible to select and use only the magnetic field signals measured by at least two magnetic sensors located in places where there is a less reduction of the magnetic field to be detected under the influence of the distance and the orientation, in spite of a detection environment or a condition for disposing magnetic sensors. By means of using the tiny permanent magnetic or the ferromagnetic body wherein the generated magnetic field strength is small, this makes it much easier to insert them into the body cavity, and it can minimize the measurement errors caused by the sensors thereby further improving a detecting accuracy of the position and the orientation of the insertion portion.

Moreover, though a Hall element or a magnetic inductance effect element or the like may be used as the magnetic sensors which is the magnetic field detecting means in the present invention, it is preferable to use a magneto-impedance effect element. The magneto-impedance effect element is easily downsized. Furthermore, through a skin effect of a high magnetic permeability magnetic body such as an amorphous wire, the magneto-impedance effect wherein the impedance of the magnetic body is sensitively changed by the outside magnetic field is used, thereby obtaining a sensitive measuring accuracy.

The medical insertion tool of the present invention may be any one selected from among indwelling tools inside the body cavity, including a guide wire, an endoscope or a drainage tube, a biliary stent, a high calorie transfusion tube as well as a catheter. Especially, in mainly a treatment and a diagnosis of brain infarction, a cerebral aneurysm or the like, the insertion tool is used to be inserted in a narrow and complicatedly wound blood vessel inside the skull. So, it is applicable for detecting the position and orientation of the catheter, which needs to be downsized.

As mentioned above, the present invention provides the medical insertion tool wherein the permanent magnet or the ferromagnetic body functioning as the magnetic field generating means is attached to the insertion portion of the medical insertion tool in order to detect the position and the orientation of the medical insertion tool inserted inside the body cavity. So, as compared with the prior art wherein a magnetic sensor functioning as the magnetic filed detecting means is attached to the insertion portion, it is not necessary to dispose electric conductors for supplying a sensor driving power and for fetching the detecting magnetic field signal, such as a coaxial cable along the insertion portion. This can achieve downsizing and lightening the insertion portion of the medical insertion tool. As a result, the present invention makes it possible to insert the insertion portion smoothly and easily into even a narrow body cavity such as a blood vessel inside a skull, without having a bad influence on a vital tissue. Moreover, it is not necessary to apply an electric current to the insertion portion of the medical insertion tool to be inserted in the body cavity. This can make S/N ratio great while minimizing a magnetic field noise which is a cause of measurement error of the magnetic sensor. Plural magnetic sensors are disposed, the magnetic sensors have triaxial directivity to the magnetic field t be detected. From among the plural magnetic sensors, we select the sensor located in a place where a distance between the permanent magnet or the ferromagnetic body and the magnetic sensor is short, and where an influence of the orientation is less and the magnetic field to be detected is great. Then, the magnetic signals measured by the abovementioned sensor are selectively used. Consequently, influence of the distance and the orientation can be minimized to decrease the measurement error, thereby making it possible to detect the three-dimensional position and orientation of the insert on portion of the predetermined medical insertion tool accurately.

### Brief Description of the Drawings

Fig. 1 is a schematic structural view of a whole of a system for detecting the position and the orientation of the tip of the catheter, which illustrates an embodiment wherein the catheter is used as a medical insertion tool to be inserted into the body cavity according to the present invention.

Fig. 2 is an enlarged schematic structural view of the catheter.

Fig. 3 is a block structural view of the magnetic field measurement signal processing circuit connected to a triaxial MI sensor accompanying the catheter.

Fig. 4 is a conceptual view illustrating a state wherein the position and the orientation of the tip of the insertion portion is detected when non-craniotomy minimally invasive treatment is performed through the catheter.

### Detailed Description of the Preferred Embodiment

Hereinafter, an embodiment of the invention will be described with reference to drawings:

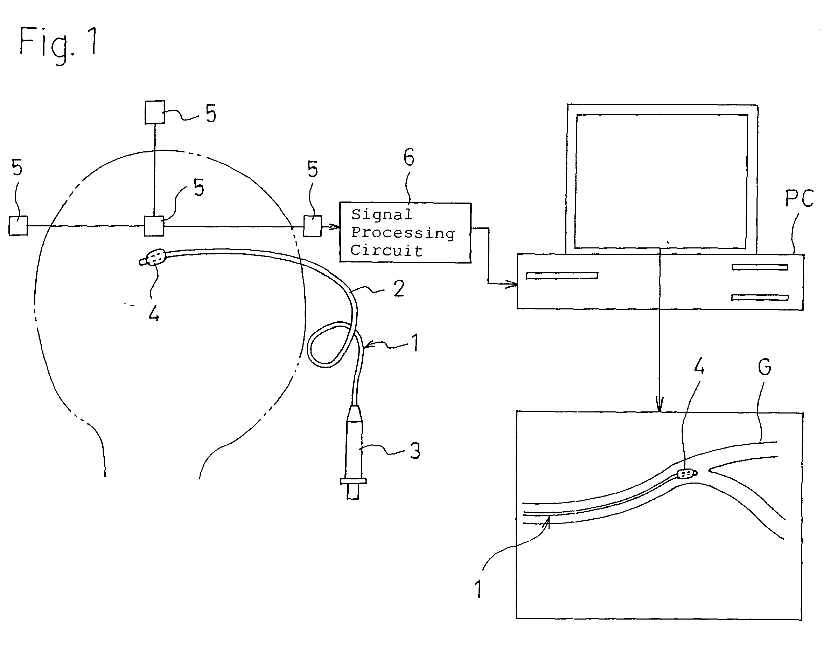

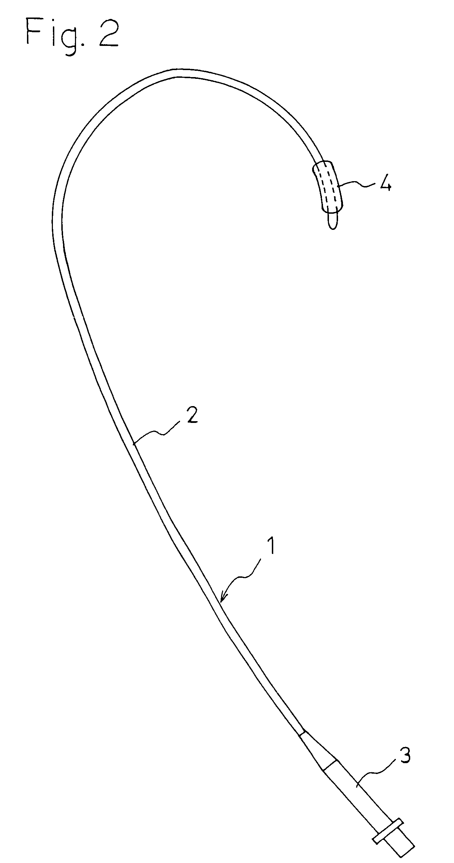

Fig. 1 is a schematic structural view of a device for detecting a position and an orientation of a medical insertion tool inside a body cavity, according to the present invention. The embodiment is applied to a catheter 1 as the medical insertion tool to be inserted into the body cavity. As shown in Fig. 2, the catheter 1 includes a narrow tubular insertion portion 2 whose diameter is 100 µ m ~ 1mm, that is, can be inserted into a blood vessel (i. e., the body cavity) , and an operation portion 3 attached to an base end portion of the insertion portion 2.

A permanent magnet 4, which functions as a magnetic field generating means, is fixedly attached to a tip of the insertion portion 2 in such a catheter 1. The permanent magnet 4 can generate a magnetic field without applying an electric current to a conductor. As the permanent magnet 4, columnar NeFeB magnet or the like whose diameter and length are respectively 2mm and 5mm, and whose surface magnetic flux density is substantially 330mT, are used. The permanent magnet 4 is fixed to the insertion portion 2 in a settled orientation. A semi-rigid ferromagnetic body instead of the permanent magnet 4 may be used.

On the other hand, a magnetic sensor 5, which functions as a magnetic field detecting means for detecting the magnetic field to be generated from the permanent magnet 4 inserted into the blood vessel, is arranged separately from the catheter 1. The magnetic sensor 5 is an MI sensor having triaxial directivity (hereinafter called "triaxial MI sensor") by means of amorphous extreme thin line and magneto-impedance effect element. The triaxial MI sensor means a triaxial directivity-magnetic sensor which is formed by combining plural magnetic sensors respectively having uniaxial directivity. Plural triaxial magnetic sensors 5 are disposed outside the body cavity.

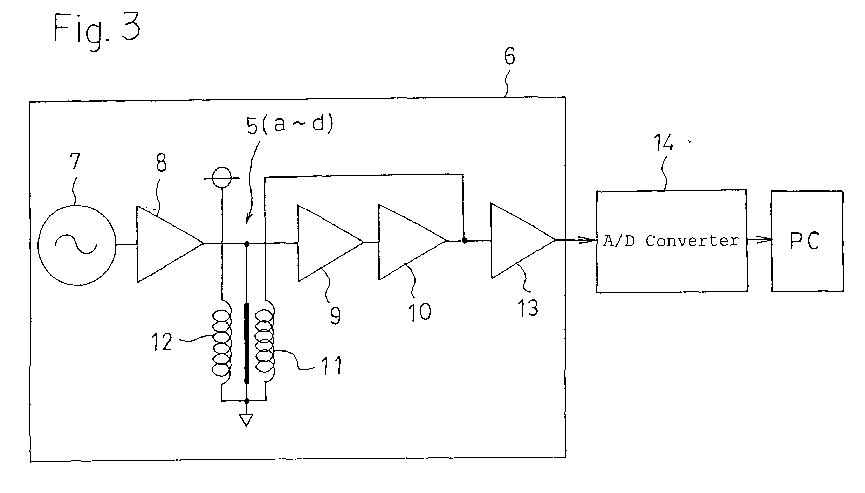

These triaxial MI sensors 5 are connected to an exclusive magnetic field measurement signal processing circuit 6. As shown in Fig. 3 illustrating one sensor having uniaxial directivity, the magnetic field measurement signal processing circuit 6 has a structure wherein a high-frequency exciting current is applied to the MI sensor 5 through an oscillator 7 and a rectification circuit 8, and the outside magnetic field generated from the permanent magnet 4 is applied thereto , whereby a modulated wave wherein the high-frequency exciting current was amplitude-modulated is ratified in an operation- detecting wave circuit 9, thereby demodulating the wave so as to detect the modulated wave, i.e., the outside magnetic field, and a negative feedback signal synchronizing with the outside magnetic field is generated through an amplifier 10, thereby feeding back the negative feed back signal to a current which is sent to a bias magnetic field generating coil 12 through a coil 11, and biasing it, so as to obtain a sensor output which has an excellent linear property and wherein a phase was discriminated.

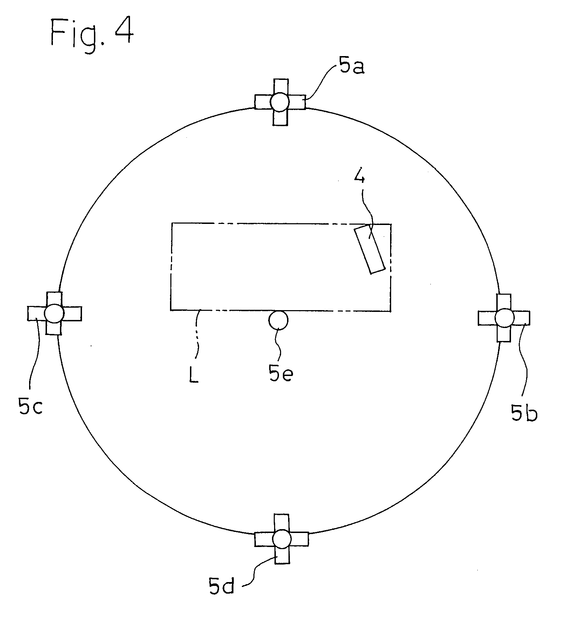

Next, we will show a method of actually detecting a position and an orientation of the tip of the insertion portion 2 when a cerebral aneurysm or the like is treated or diagnosed from an inside of the blood vessel by means of the catheter 1 having the above structure, that is non-craniotomy minimally invasive treatment is performed. In this case, as shown in Fig. 4, five triaxial MI sensors 5 (a to e) are disposed around a scope L to be detected in a head portion of a human body, that is, four triaxial MI sensors 5 are equally spaced around the scope L and one triaxial MI sensor 5 is disposed over the head portion. In this state, when the insertion portion 2 of the catheter 1 is inserted into the blood vessel of the head portion, the magnetic field generated from the permanent magnet 4 attached to the tip of the insertion portion 2 is distributed in the air at an outer periphery of the head of the human body (i.e., the outside of the body cavity) in a state wherein the magnetic field outside the body cavity has substantially the same strength as one inside the head of the human body (i. e., the body cavity) . The magnetic field distributed in the air is measured by the triaxial MI sensors 5 (a to e), and the measurement signal is processed in the exclusive signal processing circuit 6, thereby obtaining the sensor output.

Now, the sensor outputs finally required for detecting the position and orientation of the tip of the insertion portion 2 of the catheter 1 is six, that is space coordinates (3 degrees of freedom) and orientation angle (3 degrees of freedom) of the permanent magnet 4. For example, in case of Fig. 4, from among the triaxial MI sensors 5 (a to e) whose total number is five, three triaxial MI sensors 5 (c to e) are not used, because they have disadvantages wherein the permanent magnet 4 cannot approach them owing to detecting environment and sensor installation limiting condition or the like with the result that S/N ration is small and measurement error is increased. As a result, only the magnetic field measurement signals from two triaxial MI sensors 5 (a, b) which are close to the permanent magnet 4 are used, thereby making it possible to finally obtain six sensor outputs whose S/N ratios are big enough though a tiny permanent magnet 4, wherein the generatedmagnetic field strength is small, is used.

Outputs of two triaxial MI sensors 5 (a, b) thus obtained are amplified in an amplification circuit 13. After that, they are input to a computer PC through an A/D converter 14. Data on a map made by previously gathering data about a position and an angle is compared with actual detecting data from the triaxial MI sensors 5 (a, b) by means of an well-known mapping method, thereby making it possible to accurately detect the three-dimensional position and orientation of the permanent magnet 4, namely, the tip of the insertion portion 2 of the catheter 1. Moreover, on the basis of such detecting information, the catheter 1 is indicated by superimposing it on a three-dimensional blood vessel image G input to the computer PC through CT and MRI previous to an operation. This can attain a predetermined medical object of surely and easily inserting the insertion portion 2 in the direction to be aimed, while confirming safely and surely'the position and the orientation of the tip of the insertion portion 2 of the catheter 1, without unpleasantness caused by an operation performed in a state of wearing a protector for avoiding a bad influence on the human body and X-rays exposure owing to fluoroscopy.

Though the above embodiment was described about a case for applying the device to the catheter as the medical insertion tool, it may be used as a guide wire and an endoscope except the catheter. Moreover, it is applicable for detecting the position and the direction of the tip of an indwelling tool *in vivio* such as a drainage tube (ERBD tube) for forming a discharge passage for body fluid, a biliary stent, a high calorie transfusion tube, and a probe for thermotherapy in a vital tissue.

Moreover, the permanent magnet 4 as the magnetic field generating means may be disposed not only on the tip of the insertion portion 2 of the catheter 1, but also on a number of longitudinal positions thereof. Furthermore, by arranging a low pass filter (LPF) for decreasing alternating current magnet field noise occurred owing to peripheral devices, in the magnetic field measurement signal processing circuit 6, a measurement error is further decreased. This can enhance a detecting accuracy.

## Claims

1. A device for detecting a position and an orientation of an insertion portion of a medical insertion tool inside a body cavity by means of a magnetic field generating means and a magnetic field detecting means **characterized in that**
the magnetic field generating means is attached to the insertion portion of the medical insertion tool; the magnetic field generating means is made of a permanent magnet or a ferromagnetic body which can generate a magnetic field without applying an electric current to a conductor; and the magnetic field detecting means is disposed outside the body cavity, and the magnetic field detecting means including plural magnetic sensors having triaxial directivity to the magnetic field to be detected, each of the magnetic sensors having triaxial directivity being formed by combining plural sensors respectively having uniaxial directivity.

2. A device for detecting a position and an orientation of an insertion portion of a medical insertion tool inside a body cavity according to claim 1, wherein the magnetic field detecting means includes at least three magnetic sensors having triaxial directivity which are equally spaced around a scope to be detected.

3. A device for detecting a position and an orientation of an insertion portion of a medical insertion tool inside a body cavity according to claim 1 or 2, wherein the magnetic sensor of the magnetic field detecting means is a magneto-impedance effect element.

4. A device for detecting a position and an orientation of an insertion portion of a medical insertion tool inside a body cavity according to any one of claims 1 to 3, wherein the medical insertion tool is selected from among indwelling tools inside the body cavity such as a catheter, a guide wire, an endoscope or a drainage tube, a biliary stent, a high calorie transfusion tube.

5. A method of detecting a position and an orientation of an insertion portion of a medical insertion tool inside a body cavity by means of a magnetic field generating means and a magnetic field detecting means comprising the steps of:
generating a magnetic field through the magnetic field generating means made of a permanent magnet or a ferromagnetic body attached to the insertion portion without applying an electric current to a conductor, in a state wherein the insertion portion thereof is inserted inside the body cavity,
measuring the generated magnetic field by means of the plural magnetic sensors having the triaxial directivity to the magnetic field to be detected, which are disposed outside the body cavity, each of the magnetic sensors having triaxial directivity being formed by combining plural sensors respectively having uniaxial directivity and
detecting a three dimensional position and a three dimensional orientation of the insertion portion of the medical insertion tool.

6. A method of detecting a position and an orientation of an insertion portion of a medical insertion tool inside a body cavity according to claim 5, wherein the magnetic field detecting means includes at least three magnetic sensors having triaxial directivity which are equally spaced around a scope to be detected.

7. A method of detecting a position and an orientation of an insertion portion of a medical insertion tool inside a body cavity according to claim 5 or 6, wherein a magneto-impedance effect element is used as the magnetic sensor of the magnetic detecting means.
